(19) Europäisches Patentamt
European Patent Office
Office européen des brevets

(11) EP 4 316 540 A1

(12) # EUROPEAN PATENT APPLICATION
published in accordance with Art. 153(4) EPC

(43) Date of publication:
**07.02.2024 Bulletin 2024/06**

(21) Application number: **22780970.4**

(22) Date of filing: **29.03.2022**

(51) International Patent Classification (IPC):
***A61L 33/06*** *(2006.01)* ***C08F 222/10*** *(2006.01)*
***C08F 230/08*** *(2006.01)*

(52) Cooperative Patent Classification (CPC):
**A61L 33/06; C08F 222/10; C08F 230/08**

(86) International application number:
**PCT/JP2022/015625**

(87) International publication number:
**WO 2022/210759 (06.10.2022 Gazette 2022/40)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **31.03.2021 JP 2021061681**

(71) Applicant: **TOYOBO CO., LTD.**
**Osaka-shi, Osaka 530-0001 (JP)**

(72) Inventors:
- **KAWAKATSU, Yuta**
  **Osaka-shi, Osaka 530-8230 (JP)**
- **NAKAYAMA, Shohei**
  **Otsu-shi, Shiga 520-0292 (JP)**

(74) Representative: **Vossius & Partner Patentanwälte Rechtsanwälte mbB**
**Siebertstrasse 3**
**81675 München (DE)**

(54) **ANTITHROMBOTIC MATERIAL**

(57) An object of the present invention is to provide a surface treatment agent that is excellent in antithrombogenicity and biocompatibility and has higher hydrophilicity compared to conventional medical materials. The present invention is an antithrombogenic material containing a (meth)acrylate copolymer consisting of a hydrophobic (meth)acrylate and a hydrophilic (meth)acrylate, in which the hydrophobic (meth)acrylate is a silicone (meth)acrylate and/or an alkyl (meth)acrylate, the (meth)acrylate copolymer having a residual monomer content of 4,000 ppm or less, a reduced viscosity ($\eta$sp/c) of 0.18 dl/g or more and 3.00 dl/g or less, and being water-insoluble, and being a viscous liquid at room temperature.

EP 4 316 540 A1

## Description

TECHNICAL FIELD

The present invention relates to an antithrombogenic material that confers blood compatibility to a medical device. More specifically, the present invention relates to an antithrombogenic material including a (meth)acrylate copolymer containing a hydrophobic (meth)acrylate and a hydrophilic (meth)acrylate, the hydrophobic (meth)acrylate including at least a silicone (meth)acrylate, and the (meth)acrylate copolymer being water-insoluble, and being a viscous liquid at room temperature.

BACKGROUND ART

In recent years, studies on medical devices using various polymer materials have proceeded, and such devices are expected to be used for blood filters, artificial kidneys, plasma separation, catheters, artificial lungs, artificial blood vessels, anti-adhesion films, artificial skin, and the like. In this case, the medical devices are required to be biocompatible because the synthetic material, which is a foreign substance for the living body, is used in contact with living tissues or blood.

When a medical device is used as a material in contact with blood, the following three factors are important for biocompatibility: (a) suppression of the blood coagulation system; (b) suppression of adhesion and activation of platelets; and (c) suppression of activation of the complement system. In particular, when the medical device is used as a material that has a relatively short contact time with the blood, such as a medical device for extracorporeal circuit (e.g., an artificial kidney or plasma separation membrane), it is particularly important to suppress the activation of platelets and the complement system described in (b) and (c) above, because, in general, anticoagulants such as heparin and sodium citrate are simultaneously used with the device.

The applicant has studied the material suitable for the above use and has filed a patent application for an antithrombogenic material containing a (meth)acrylate copolymer consisting of an alkyl (meth)acrylate, a silicone (meth)acrylate, and a methoxy polyethylene glycol (meth)acrylate, where the (meth)acrylate copolymer has a number-average molecular weight of 2,000 to 200,000, is water-insoluble, and a viscous liquid at room temperature, soluble in any of the alcohols having 1 to 6 carbon atoms, and has a viscosity of 0.5 to 10,000 Pa s at 37°C (PTL 1).

CITATION LIST

PATENT LITERATURE

PTL 1: Japanese Patent No. 4793700

SUMMARY OF INVENTION

TECHNICAL PROBLEM

An object of the present invention is to provide an antithrombogenic material that can be used as a material having a relatively long contact time with blood compared to conventional medical materials, and that has further enhanced durability against blood contact.

SOLUTION TO PROBLEM

The present inventors have found that adjusting a residual monomer content and a reduced viscosity to particular ranges in a copolymer obtained by copolymerizing an alkyl (meth)acrylate, a methoxy polyethylene glycol (meth)acrylate, and a silicone (meth)acrylate, and applying the copolymer to a medical device can confer antithrombogenicity for a longer period than in the past, and have completed the present invention. That is, the present invention has the following configurations.

(1) An antithrombogenic material comprising a (meth)acrylate copolymer having a weight-average molecular weight of 50,000 or more and 1,500,000 or less, the (meth)acrylate copolymer being obtained by copolymerizing an alkyl (meth)acrylate unit represented by the following Formula 1, a silicone (meth)acrylate unit represented by the following Formula 2, and a methoxy polyethylene glycol (meth)acrylate unit represented by the following Formula 3 at a molar ratio of 80-20/10-0.01/10-79.99, wherein the (meth)acrylate copolymer is water-insoluble, the water-insoluble means that, when the (meth)acrylate copolymer is left still for 30 days in a physiological saline at 37°C at 99 mass% with

respect to 1 mass% of the copolymer, the mass reduction rate of the copolymer is 1 mass% or less, the (meth)acrylate copolymer is a viscous liquid at room temperature, the (meth)acrylate copolymer is soluble in any of alcohols having 1 to 6 carbon atoms, a residual monomer content is 4,000 ppm or less, and a reduced viscosity (ηsp/c) is 0.18 dl/g or more and 3.00 dl/g or less,

[Formula 4]

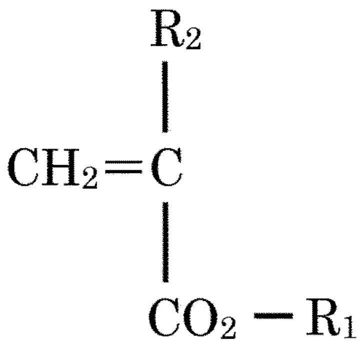

[1]

wherein, $R^1$ represents an alkyl group having 8 to 12 carbon atoms, $R^2$ represents a hydrogen atom or a methyl group,

[Formula 5]

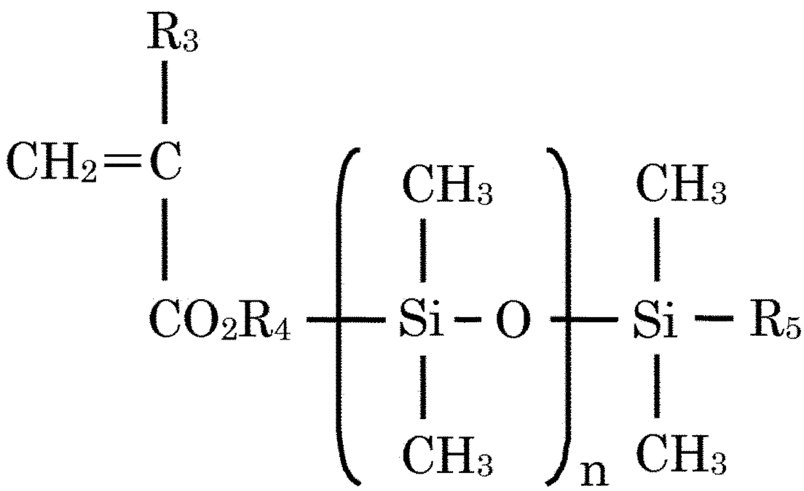

[2]

wherein, $R^3$ is a hydrogen atom or a methyl group, $R^4$ is an alkylene group having 1 to 6 carbon atoms, $R^5$ is an alkyl group having 1 to 6 carbon atoms, and n is an integer of 1 to 30,

[Formula 6]

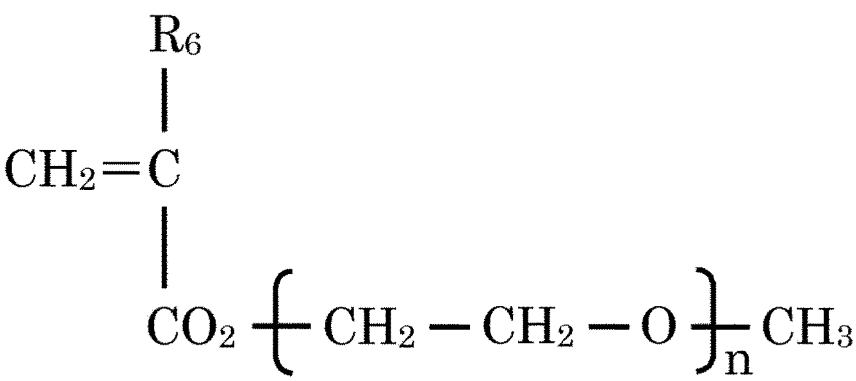

[3]

wherein $R^6$ represents a hydrogen atom or a methyl group, and n represents an integer of 2 to 4.

(2) A medical device comprising the antithrombogenic material according to (1).

ADVANTAGEOUS EFFECTS OF INVENTION

The antithrombogenic material according to the present invention is a material having excellent biocompatibility and high durability to the blood contact. In addition, since the physical properties of the material are viscous and insoluble in water, it will not easily elute into the blood when the antithrombogenic material carried on a medical device comes into contact with the blood, and the medical device can maintain the antithrombogenicity for a long time. Furthermore, since the material contains a silicone (meth)acrylate as the hydrophobic (meth)acrylate, the effect of inhibiting the hydrolysis of the (meth)acrylate copolymer can be expected due to the water-repellent action.

DESCRIPTION OF EMBODIMENTS

In the present invention, it is preferred that the (meth)acrylate copolymer containing a hydrophobic (meth)acrylate and a hydrophilic (meth)acrylate has the durability to the blood contact and is in a viscous liquid form at room temperature. Here, having the durability to the blood contact means that when a (meth)acrylate copolymer is immersed in an alcohol immersion treatment solution for 16 hours at room temperature, the (meth)acrylate copolymer remains at least a certain amount and exhibits antithrombogenicity. When a (meth)acrylate copolymer remains at least a certain amount after being immersed in an alcohol immersion treatment solution at room temperature for 16 hours, it can be determined that the (meth)acrylate copolymer has (maintains) sufficient antithrombogenicity even when contacted with blood at 37°C for 30 days. Furthermore, since the (meth)acrylate copolymer is a liquid but viscous at room temperature, there is an advantage that, even when the (meth)acrylate copolymer is used for coating a medical device or the like, the elution into the blood can be suppressed.

In the present invention, the hydrophilic (meth)acrylate preferably includes a methoxy polyethylene glycol (meth)acrylate represented by the following Formula 3. In the following Formula 3, it is preferable that the number of ethylene oxide units is 2 to 10, more preferably, 2 to 5. Specific examples include methoxy diethylene glycol (meth)acrylate, methoxy triethylene glycol (meth)acrylate, methoxy tetraethylene glycol (meth)acrylate, methoxy pentaethylene glycol (meth)acrylate, methoxy hexaethylene glycol (meth)acrylate, methoxy heptaethylene glycol (meth)acrylate, methoxy octaethylene glycol (meth)acrylate, methoxy nonaethylene glycol (meth)acrylate, and methoxy decaethylene glycol (meth)acrylate. When the number of repeat units is too large and the hydrophilicity becomes too high, elution of the copolymer into blood increases, and may cause easy peeling from the medical material. Thus, methoxy tetraethylene glycol (meth)acrylate having 4 ethylene oxide repeat units or methoxy triethylene glycol (meth)acrylate having 3 ethylene oxide repeat units are further preferred, and methoxy triethylene glycol (meth)acrylate having 3 ethylene oxide repeat units particularly preferred.

[Formula 7]

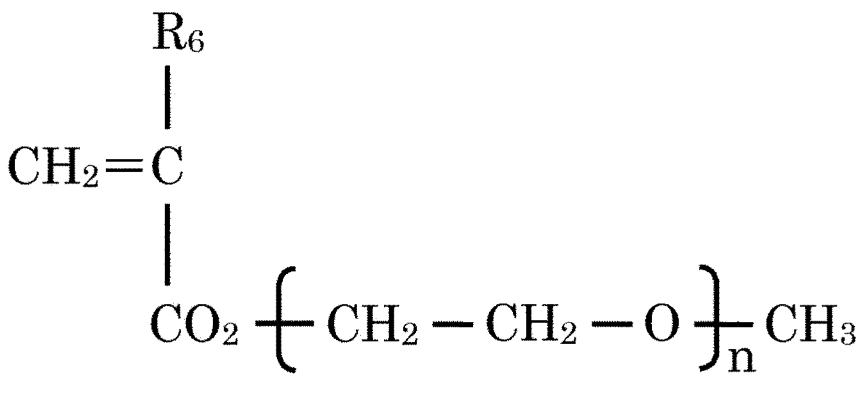

[3]

wherein, $R^6$ represents a hydrogen atom or a methyl group, and n represents an integer of 2 to 4.

In the present invention, the hydrophobic (meth)acrylate preferably includes a silicone (meth)acrylate represented by the following Formula 2. The silicone (meth)acrylate preferably includes a silicone (meth)acrylate having 1 to 50 dimethylsiloxane repeat units. When the number of repeat units is too large, the resulting copolymer may be too viscous and difficult to handle. When the number of repeat units is too small, the viscosity may be too low and the

resulting copolymer may easily disappear from the coating surface of the medical device or the like. Thus, the number of dimethylsiloxane repeat units is more preferably 1 to 20.

[Formula 8]

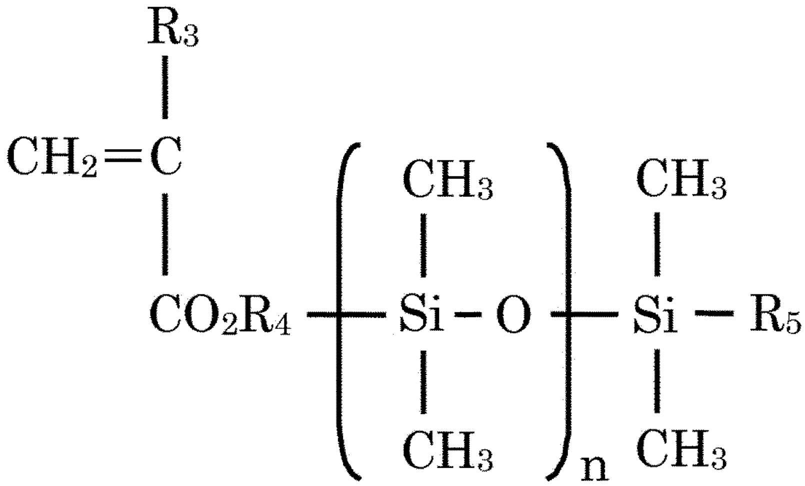

[2]

wherein, $R^3$ is a hydrogen atom or a methyl group, $R^4$ is an alkylene group having 1 to 6 carbon atoms, $R^5$ is an alkyl group having 1 to 6 carbon atoms, and n is an integer of 1 to 30.

In the present invention, the hydrophobic (meth)acrylate may contain other hydrophobic (meth)acrylates other than the silicone (meth)acrylate. The other hydrophobic (meth)acrylates are not particularly limited, but as an example, a hydrophobic (meth)acrylate represented by the following Formula 1 may preferably be added. In the following Formula 1, $R^1$ is preferably a group having 8 to 20 carbon atoms, more preferably 8 to 12 carbon atoms. Specific examples of such hydrophobic (meth)acrylates include normal hexyl (meth)acrylate, cyclohexyl (meth)acrylate, phenyl (meth)acrylate, benzyl (meth)acrylate, heptyl (meth)acrylate, octyl (meth)acrylate, 2-ethylhexyl (meth)acrylate, nonyl (meth)acrylate, decyl (meth)acrylate, lauryl (meth)acrylate, myristyl (meth)acrylate, palmityl (meth)acrylate, and stearyl (meth)acrylate, and 2-ethylhexyl (meth)acrylate and lauryl (meth)acrylate are particularly preferred from the viewpoint of cost and performance.

[Formula 9]

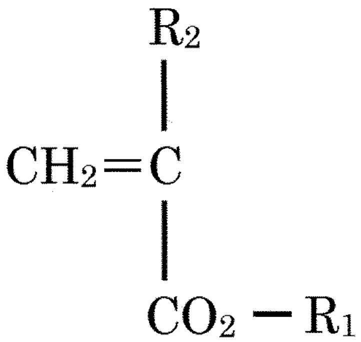

[1]

wherein, $R^1$ represents an alkyl group having 8 to 12 carbon atoms, $R^2$ represents a hydrogen atom or a methyl group.

Specific examples of the water-insoluble (meth)acrylate copolymer according to the present invention include, but are not limited to, a (meth)acrylate copolymer consisting of an alkyl (meth)acrylate unit represented by Formula 1, a silicone (meth)acrylate unit represented by the following Formula 2, and a methoxy polyethylene glycol (meth)acrylate unit represented by the following Formula 3 at a molar ratio of 80-20/10-0.01/10-79.99, such as a silicone (meth)acrylate-methoxy diethylene glycol (meth)acrylate copolymer, a silicone (meth)acrylate-methoxy triethylene glycol (meth)acrylate copolymer, a silicone (meth)acrylate-methoxy tetraethylene glycol (meth)acrylate copolymer, a silicone (meth)acrylate-normalhexyl (meth)acrylate-methoxy diethylene glycol (meth)acrylate copolymer, a silicone (meth)acrylate-normalhexyl (meth)acrylate-methoxy triethylene glycol (meth)acrylate copolymer, a silicone (meth)acrylate-normalhexyl (meth)acrylate-methoxy tetraethylene glycol (meth)acrylate copolymer, a silicone (meth)acrylate-cyclohexyl (meth)acrylate-methoxy diethylene glycol (meth)acrylate copolymer, a silicone (meth)acrylate-cyclohexyl (meth)acrylate-methoxy triethylene glycol (meth)acrylate copolymer, a silicone (meth)acrylate-cyclohexyl (meth)acrylate-methoxy tetraethylene glycol (meth)acrylate copolymer, a silicone (meth)acrylate-phenyl (meth)acrylate-methoxy diethylene glycol (meth)acrylate copolymer, a silicone (meth)acrylate-phenyl (meth)acrylate-methoxy triethylene glycol (meth)acrylate copolymer, a silicone (meth)acrylate-phenyl (meth)acrylate-methoxy tetraethylene glycol (meth)acrylate copolymer, a silicone (meth)acrylate-n-octyl (meth)acrylate-methoxy diethylene glycol (meth)acrylate copolymer, a silicone (meth)acrylate-n-octyl (meth)acrylate-methoxy triethylene glycol (meth)acrylate copolymer, a silicone (meth)acrylate-n-octyl (meth)acrylate-methoxy tetraethylene glycol (meth)acrylate copolymer, a silicone (meth)acrylate-2-ethylhexyl (meth)acrylate-methoxy diethylene glycol (meth)acrylate copolymer, a silicone (meth)acrylate-2-ethylhexyl (meth)acrylate-methoxy triethylene glycol (meth)acrylate copolymer, a silicone (meth)acrylate-2-ethylhexyl (meth)acrylate-methoxy tetraethylene glycol (meth)acrylate copolymer, a silicone (meth)acrylate-lauryl (meth)acrylate-methoxy diethylene glycol (meth)acrylate copolymer, a silicone (meth)acrylate-lauryl (meth)acrylate-methoxy triethylene glycol (meth)acrylate copolymer, a silicone (meth)acrylate-lauryl (meth)acrylate-methoxy tetraethylene glycol (meth)acrylate copolymer, a silicone (meth)acrylate-n-nonyl (meth)acrylate-methoxy diethylene glycol (meth)acrylate copolymer, a silicone (meth)acrylate-n-nonyl (meth)acrylate-methoxy triethylene glycol (meth)acrylate copolymer, a silicone (meth)acrylate-n-nonyl (meth)acrylate-methoxy tetraethylene glycol (meth)acrylate copolymer, silicone (meth)acrylate-n-decyl (meth)acrylate-methoxy diethylene glycol (meth)acrylate copolymer, a silicone (meth)acrylate-n-decyl (meth)acrylate-methoxy triethylene glycol (meth)acrylate copolymer, a silicone (meth)acrylate-n-decyl (meth)acrylate-methoxy tetraethylene glycol (meth)acrylate copolymer, a silicone (meth)acrylate-stearyl (meth)acrylate-methoxy diethylene glycol (meth)acrylate copolymer, a silicone (meth)acrylate-stearyl (meth)acrylate-methoxy triethylene glycol (meth)acrylate copolymer, a silicone (meth)acrylate-stearyl (meth)acrylate-methoxy tetraethylene glycol (meth)acrylate copolymer, a silicone (meth)acrylate-lauryl (meth)acrylate-methoxy diethylene glycol (meth)acrylate copolymer, a silicone (meth)acrylate-lauryl (meth)acrylate-methoxy triethylene glycol (meth)acrylate copolymer, a silicone (meth)acrylate-lauryl (meth)acrylate-methoxy tetraethylene glycol (meth)acrylate copolymer, a silicone (meth)acrylate-myristyl (meth)acrylate-methoxy diethylene glycol (meth)acrylate copolymer, a silicone (meth)acrylate-myristyl (meth)acrylate-methoxy triethylene glycol (meth)acrylate copolymer, and a silicone (meth)acrylate-myristyl (meth)acrylate-methoxy tetraethylene glycol (meth)acrylate copolymer. When the content of the hydrophobic (meth)acrylate is too small, the copolymer tends to be dissolved in the blood or the like, and when the content of the hydrophobic (meth)acrylate is too large, the blood compatibility of the hydrophilic (meth)acrylate may not be sufficiently exhibited. Thus, the molar ratio is more preferably 80-50/5-0.01/15-49.99, further preferably 77-55/5-0.01/18-44.99, and even further preferably 73-57/5-0.01/22-42.99.

Indicators representing a molecular weight of a copolymer include a number-average molecular weight and a weight-average molecular weight. Since the weight-average molecular weight more relates to stability and adhesion, the weight-average molecular weight is selected as the indicator in the present invention. When the weight-average molecular weight is too small, not only can the copolymer easily elute into the blood, but the strength and stability of the coating film may be lost. In addition, since a larger weight-average molecular weight leads to a higher viscosity in preparing the coating solution, an additional effect of improving the adhesion to the coating substrate will be exerted. It is thus more preferred that the weight-average molecular weight of the (meth)acrylate copolymer is 60,000 or more. Further, it is preferred that the weight-average molecular weight of the (meth)acrylate copolymer is 1,500,000 or less because the workability in coating a medical device or the like with the (meth)acrylate copolymer is improved. More preferably, the weight-average molecular weight of the (meth)acrylate copolymer is 1,000,000 or less, further preferably 500,000 or less. Here, the weight-average molecular weight is the sum of the molecular weights of all molecules divided by the sum of the weights of the molecules, and one of the indicators for the properties of a polymer.

The setting of the weight-average molecular weight of the (meth)acrylate copolymer to 50,000 or more and 1,500,000 or less is also technically very important for achieving specific technical issues related to purification of the copolymer, handling of the treatment solution, adaptability to medical devices, stability of the coating film, and the like.

Methods for measuring the weight-average molecular weight include a terminal group quantification method, an osmotic method, a vapor pressure osmometry method, a vapor pressure drop method, a freezing point drop method, a boiling point rise method, a gel permeation chromatography (GPC) method, and the like, but in the present invention, the gel permeation chromatography (GPC) method, which is a conventional method, is employed due to the ease of operation.

The reduced viscosity ($\eta sp/c$) of the (meth)acrylate copolymer is preferably 0.18 dl/g or more and 3.00 dl/g or less in the present invention. By using a copolymer (antithrombogenic material) having such a viscosity range, when coated to a medical device such as an artificial cardiopulmonary circuit, a catheter, or the like, the copolymer has excellent adhesion to the medical device, and the antithrombogenicity can be maintained in the long-term use. The more preferred range of the reduced viscosity is 0.18 dl/g or more and 1.50 dl/g or less, still more preferably 0.18 dl/g or more and 0.50 dl/g or less.

Although the hydrophobic (meth)acrylate in the present invention does not necessarily include a silicone (meth)acrylate, silicone has an advantage of capability of exhibiting stable properties in various temperature ranges due

to the excellent heat resistance, cold resistance, and low glass transition temperature (Tg) as can be seen from its basic backbone. In addition, due to the large binding energy, it has advantages of acid and alkali resistance and high chemical stability. Furthermore, it has excellent copolymerization properties with (meth)acrylate monomers, and can be preferably used as a raw material of the (meth)acrylate copolymer according to the present invention. Silicone (meth)acrylate is also recognized as a material with high safety for living organisms, and has been used in contact lens materials in recent years. Thus, a large content of silicone (meth)acrylate in the antithrombogenic material may not raise any problems, and a silicone (meth)acrylate-methoxy polyethylene glycol (meth)acrylate copolymer may be used. However, the current raw material price of silicone (meth)acrylate is relatively high, and using silicone (meth)acrylate alone as the hydrophobic (meth)acrylate may be disadvantageous in terms of cost-effectiveness. Considering the performance, quality, cost, or the like comprehensively, it can be said that the upper limit of the amount of silicone (meth)acrylate added is sufficiently 50 mass% or less, more preferably 40 mass% or less, further preferably 35 mass% or less. The silicone (meth)acrylate may also be mixed with the other alkyl (meth)acrylates described above. When the content of the silicone (meth)acrylate is too small, the hydrolysis may progress during storage and the long-term stability of the antithrombogenic material may be reduced. Thus, the content of silicone (meth)acrylate in the hydrophobic (meth)acrylate is more preferably 0.1 mass% or more, further preferably 0.5 mass% or more, and still more preferably 1.0 mass% or more.

The (meth)acrylate copolymer may be a copolymer in which the monomer represented by Formula 2 and the monomer represented by Formula 3 are alternately arranged. When analyzed from the total amount, the (meth)acrylate copolymer may also be a copolymer consisting of a segment or block consisting of a hydrophobic monomer and a segment or block consisting of a hydrophilic monomer. It is also conceivable that the (meth)acrylate copolymer may have a complex structure, such as a so-called microphase separation structure or mosaic pattern structure in which the segment or block consisting of a hydrophobic monomer serves to fix the segment or block consisting of a hydrophilic monomer. In any case, although the large or small molecular weight of the copolymer and the type and characteristics of the hydrophilic monomer have some influence, a slightly larger amount of the hydrophobic monomer can suppress the elution of the segment or block consisting of the hydrophilic monomer of the copolymer. It is believed that the slightly larger amount of the hydrophobic segment also serves to increase the affinity with hydrophobic medical devices, and it can be assumed that it will be advantageous in the fixation to medical devices as a coating film. Although it is currently not able to accurately verify the performance regarding the presence or absence of a segment and its affinity state based on technical evidence, the copolymer is a polymeric material having a biologically friendly affinity.

The homopolymer of methoxy polyethylene glycol (meth)acrylate is highly hydrophilic and thus excellent in blood compatibility. However, because it is water-soluble, it has a problem of gradual elution when exposed to blood or the like for a long time. The present inventors have intensively studied about materials having not only excellent blood compatibility, but also durability for long-term use. As a result, they have found that a copolymer obtained by imparting a moderate hydrophobicity to prevent elution into the blood or the like and flexibility to prevent physical film peeling of the coating film can solve the problem.

In this way, the copolymer contained in a treatment solution is substantially composed of two types of monomer components serving so-called different bifacial interface functions, which are composed of a hydrophilic monomer, segment, or block having functions like antithrombogenicity and elution prevention to the blood and a hydrophobic monomer, segment, or block having functions like affinity and fixation to the medical device. However, it also seems that the monomers, segments, or blocks composing the copolymer are complement each other in the molecular structure to form a molecular bond or structure that is stable to the elution, dispersion, and the like.

In the present invention, the (meth)acrylate copolymer is preferably soluble in any alcohols having 1 to 6 carbon atoms. It is more preferred that the (meth)acrylate copolymer is soluble in alcohols having 1 to 3 carbon atoms because the drying after coating is easily performed. Here, "soluble" refers to that, when 1 g of the (meth)acrylate copolymer is immersed in 10 ml of the alcohol at 25°C, at least 90 mass% of the (meth)acrylate copolymer is dissolved within 16 hours at room temperature.

In the present invention, the antithrombogenic material consisting of a (meth)acrylate copolymer may contain a substance such as an antimicrobial substance. The antimicrobial substance is not particularly limited, and examples thereof include antibiotics such as ampicillin, nafcillin, amoxicillin, oxacillin, azlocillin, penicillin G, carbenicillin, penicillin V, dicloxacillin, pheneticillin, floxacillin, piperacillin, mecillinam, sulbenicillin, methicillin, ticarcillin, mezlocillin, cefaclor, cephalotin, cefadroxyl, cephapyrin, cefamandole, cephradine, cefatrizine, cefsulodin, cefazolin, ceftazidime, ceforanide, ceftriaxone, cefoxitin, cefuroxime, cefacetrile, latamoxef, cefalexin, amikacin, neomycin, dibekacin, kanamycin, gentamicin, netilmicin, kanamycin, tobramycin, amphotericin B, novobiocin, bacitracin, nystatin, clindamycin, polymyxin, colistin, rovamycine, erythromycin, streptomycin, spectinomycin, lincomycin, vancomycin, chlortetracycline, oxytetracycline, demeclocycline, rolitetracycline, doxycycline, tetracycline, and minocycline; antifungals such as amphotericin B, ketoconazole, clotrimazole, miconazole, econazole, natamycin, flucytosine, nystatin, and griseofulvin; paraoxybenzoates such as isobutyl paraoxybenzoate, isopropyl paraoxybenzoate, ethyl paraoxybenzoate, butyl paraoxybenzoate, and propyl paraoxybenzoate; biguanide compounds such as chlorhexidine; compounds having surface activity, such as benzethonium, benzalkonium, lauryl sulfate, alkyl polyaminoethyl glycine, fatty acids, and domiphene bromide; phenol

derivatives such as thymol, phenol, hexachlorophene, and resorcin; boric acid compounds such as boric acid and borax; iodine compounds such as iodine, iodoform, and povidone iodine; metals such as gold, silver, copper, and mercury, metal compounds such as thimerosal, and sulfadiazine silver; antimicrobial pigment compounds such asacrinol and methylrosaniline; and sulfa agents such as mafenide acetate, sulfadiazine, sulfisomidine, and sulfamethoxazole. These antimicrobial substances may be salt compounds such as a sodium salt, a potassium salt, a magnesium salt, a calcium salt, a hydrochloride salt, a sulfate salt, and a gluconate salt, and two or more antimicrobial substances may be used in combination.

The above antimicrobial substances can be broadly classified into water-soluble substances and poorly water-soluble substances. Examples of the water-soluble antimicrobial substances include benzalkonium chloride, povidone iodide, penicillin G potassium, and streptomycin sulfate. Examples of the poorly water-soluble antimicrobial substances include sulfadiazine silver and chlorhexidine.

In the present invention, when the antithrombogenic substance (copolymer) and a poorly water-soluble antimicrobial substance are coated on a medical device or the like, the elution of the antimicrobial substance becomes extremely small and the antimicrobial substance is sustained and the long-term antimicrobial property can be maintained, even though it is unknown whether it is because the copolymer is water-insoluble or because the water-insolubility of the copolymer and the poorly water-solubility of the antimicrobial substance function in a complementary manner. When the copolymer and a water-soluble antimicrobial substance are coated, the amount eluted is larger than when the poorly water-soluble antimicrobial substance is used because the antimicrobial substance is water-soluble even though the copolymer is water-insoluble, and the long-term antimicrobial property cannot be maintained although the strong antimicrobial property can be exhibited for a moment. For example, endovascular catheters, infusion tubes, artificial lungs, and the like are medical tools that are used continuously for one to several days, and the applications for such equipment require long-term antimicrobial properties. In addition, by the combined use of the copolymer with water-soluble and poorly water-soluble antimicrobial substances, multi-stage antimicrobial property in which strong biocidal activity is initially exhibited by the water-soluble antimicrobial substance and then the long-term antimicrobial property is exhibited by the poorly water-soluble antimicrobial substance can be imparted. When the multi-stage antimicrobial property is applied to an endovascular catheter, the water-soluble antimicrobial material elutes early and exhibits the strong antimicrobial property, thereby sterilizing the skin resident bacteria that are brought into the blood vessel upon inserting of the catheter, and reducing the risk of infection upon insertion. Then, during the catheter placement, the long-term antimicrobial property of the poorly water-soluble antimicrobial substance can prevent bacterial colonization in the catheter or propagation of bacteria infested from the insertion site, thereby reducing the risk of infection upon the catheter placement.

In the present invention, the antimicrobial substance is preferably 0.01 mass% or more and 70 mass% or less, more preferably 0.05 mass% or more and 50 mass% or less, further preferably 0.1 mass% or more and 30 mass% or less, and still further preferably 0.1 mass% or more and 10 mass% or less with respect to the mass of the antithrombogenic composition. When the content of the antimicrobial substance is too small, the antimicrobial property of the antimicrobial substance may not be sufficiently exhibited. When the content of the antimicrobial substance is too large, appearance defects of the medical tools after surface treatment such as a coating may occur, and the elution of the antimicrobial substance into the body may increase to cause local inflammation due to the eluted antimicrobial substance. Thus, the antimicrobial substance is allowed to be present on the entire surface of the medical tools, but it is preferred that the antimicrobial substance is only present near the penetration site through which the skin is inserted in terms of suppression of local inflammation.

The antithrombogenic material of the present invention may be any of a random copolymer, a block copolymer, and a graft copolymer. The copolymerization reaction itself for producing the antithrombogenic material of the present invention is not particularly limited, and known methods such as radical polymerization, ion polymerization, photopolymerization, macromer-based polymerization, and the like can be used.

A production method by radical polymerization is shown below as an example of producing the (meth)acrylate copolymer according to the present invention.

For example, a monomer, a polymerization solvent, and an initiator are added to a stirrable reactor equipped with a reflux tower, then polymerization is started by heating after nitrogen replacement, and the temperature is maintained for a certain period to promote the polymerization. It is also possible that a chain transfer agent is used during the polymerization to control the molecular weight. After completion of the polymerization, the solvent is removed from the solution to obtain a crude (meth)acrylate copolymer. Subsequently, the obtained crude (meth)acrylate copolymer is stirred in a poor solvent to perform a purification treatment. The purification treatment is repeated one to several times to increase the purity of the (meth)acrylate copolymer. The copolymer thus obtained is then dried.

Examples of the polymerization solvent used in the copolymerization include alcohols such as methanol, ethanol, and isopropyl alcohol; organic solvents such as ethyl acetate, toluene, benzene, and methyl ethyl ketone; and water. However, in terms of the solubilities of the monomers and resulting copolymer and the availability, ethyl acetate, methanol, ethanol, and the like are preferably used in the present invention. A plurality of the solvents can also be mixed to use.

The feed mass ratio of the polymerization solvents to monomers is preferably 20-90/60-10, more preferably 30-90/70-10, and further preferably 35-85/65-15. When the feed ratio is within the above range, the polymerization reaction rate can be maximized.

As the polymerization initiator, a peroxide-based radical initiator or an azo-based radical initiator, which are commonly used in radical polymerization, is used. Examples of the peroxide-based radical initiator include inorganic-based peroxides such as potassium persulfate, ammonium persulfate, and hydrogen peroxide, and organic-based peroxides such as benzoyl peroxide, t-butyl hydroperoxide, and cumene peroxide. Examples of the azo-based radical initiator include 2,2'-azobisisobutyronitrile, 2,2'-azobis(2-aminodipropane) dihydrochloride, dimethyl 2,2'-azobisbutyrate, and dimethyl 2,2'-azobis(2-methylpropionate). A redox initiator that is a combination of a peroxide-based initiator with a reducing agent can also be used. The polymerization initiator and the like are preferably added at 0.01 mass% or more and 1 mass% or less, more preferably, 0.05 mass% or more and 0.5 mass% or less, and further preferably 0.05 mass% or more and 0.3 mass% or less, with respect to the monomer in the polymerization solution. When the polymerization initiator or the like is added in the above range, a copolymer having an appropriate weight-average molecular weight can be obtained with a good monomer reaction rate.

The temperature during polymerization varies depending on the type of solvent and the type of initiator, but it is preferred that the temperature is around the 10-hour half-life temperature of the initiator. Specifically, when the initiator is used, the temperature is preferably 20°C or more and 90°C or less, more preferably, 30°C or more and 90°C or less, and further preferably 40°C or more and 90°C or less. Examples of the chain transfer agent used to control the molecular weight during polymerization include high-boiling point thiol compounds such as dodecylmercaptan, thiomalic acid, and thioglycolic acid, isopropyl alcohol, phosphorous acid, and hypophosphorous acid.

The (meth)acrylate copolymer according to the present invention is obtained by copolymerizing hydrophilic and hydrophobic monomers, and thus has both hydrophilic and hydrophobic properties. Accordingly, in the solution after the copolymerization, components of an unreacted hydrophilic monomer (methoxy polyethylene glycol (meth)acrylate), an unreacted hydrophobic monomer (silicone (meth)acrylate, and optionally alkyl (meth)acrylate), and a (meth)acrylate copolymer coexist. To isolate the water-insoluble (meth)acrylate copolymer from the mixture, the copolymer can be purified, for example, by adding dropwise the copolymer solution to a solvent that dissolves the hydrophilic monomer to perform purification, and then using a solvent that dissolves the hydrophobic monomer. In addition, the (meth)acrylate copolymer can be recovered efficiently by using a poor solvent for reprecipitation in which alcohol and water are mixed at a certain proportion. In addition, purification can be performed by repeating the following processes: in the solution after completion of the polymerization reaction, adding a poor solvent consisting of a mixture of alcohol and water at a certain proportion to a water-insoluble (meth)acrylate copolymer, and stirring them at a constant temperature to separate the (meth)acrylate copolymer, then recovering the precipitate by decanting, and adding a washing solution.

In the present invention, a poor solvent that does not dissolve the copolymer but dissolves both hydrophilic and hydrophobic monomers is preferred as a poor solvent used to purify the copolymer.

In the present invention, the alcohol used in the reprecipitation process is preferably an alcohol having 1 or more and 10 or less carbon atoms, more preferably an alcohol having 1 or more and 7 or less carbon atoms, and further preferably an alcohol having 1 or more and 4 or less carbon atoms. Specific examples of such alcohols include methanol, ethanol, 1-propanol, 2-propanol, 1-butanol, 2-butanol, 2-methoxy-1-propanol, and tertiary butanol. Among them, methanol, ethanol, 1-propanol, and 2-propanol are more preferred because the drying can be performed at low temperatures and for a short period.

In the present invention, the residual monomer content (the unreacted monomer content in the polymerization) is important because it relates to the safety. It is a matter of course that the residual monomer content in the antithrombogenic material is reduced to meet the criteria set out in the guidelines for elution from medical devices. However, the fact that setting the residual monomer content to a very low level of 4,000 ppm or less leads to an increase in the adhesion and retention of the antithrombogenic material on the surface of the medical tools is an unexpected effect. The ratio of the amount of the obtained polymer to the amount of the input monomer is expressed as a recovery rate.

When the purification treatment described above is performed once, if necessary 2 to 8 times, the water-insoluble (meth)acrylate copolymer having an unreacted monomer content of 4,000 ppm or less can be recovered at a high recovery rate of 30 mass% or more. When the content of unreacted monomers, oligomers, and polymerization residues contained in the copolymer is large, it is conceivable that they will elute into the blood and become causative substances of a shock symptom or the like in a patient. Most of the causative substances can be removed by purification treatments, but in view of the safety of patients, it is more preferred that the content of the causative substances is 3,000 ppm or less, further preferably 2,000 ppm or less, and particularly preferably 1,000 ppm or less.

The volume ratio of the crude (meth)acrylate copolymer according to the present invention to the poor solvent is preferably 1 : 1 to 1 : 20, and more preferably 1 : 3 to 1 : 10. The purification efficiency and the recovery rate can be maximized when the volume ratio is in the above range.

The temperature during purification of the crude (meth)acrylate copolymer according to the present invention is preferably 30 to 60°C, and more preferably 40 to 60°C. When the temperature during purification is in the above range,

the viscosity of the crude (meth)acrylate copolymer is reduced by heating to facilitate the separation from the poor solvent, and thus the recovery rate of the (meth)acrylate copolymer can be maximized.

In the present invention, when the recovery rate of the (meth)acrylate copolymer after the purification treatment exceeds 90 mass%, the unreacted monomer may be contained in the recovery product, and when the recovery rate falls below 20 mass%, the production efficiency is reduced. Thus, the recovery rate is preferably 20 to 90 mass%. Securing this recovery rate to 20 to 90 mass% also means that the copolymer is slightly lost or discarded, but this is necessary to prevent contamination of unreacted monomers as much as possible. In view of the specific circumstances that it is a copolymer having both hydrophilic and hydrophobic properties to apply to medical devices, such considerations must be taken for granted.

The purified copolymer is required to remove the solvent by drying for use in imparting antithrombogenicity to medical devices. The drying is performed, for example, at 60°C under reduced pressure of 1 Torr or less. The drying under reduced pressure may be continued when the copolymer is not sufficiently dried.

The antithrombogenic material of the present invention described above is water-insoluble, and can be preferably used as a surface treatment agent for medical devices or the like. As a specific aspect, it can be performed by applying the solution obtained by dissolving the obtained antithrombogenic material in an organic solvent to the surface of a substrate such as a medical device, and then removing the solvent. Examples of the method of supporting the antithrombogenic material of the present invention on the surface of the substrate include known methods, such as a coating method, a method of utilizing graft polymerization by radiation, electron rays, or ultraviolet rays, and a method of utilizing a chemical reaction with a functional group of the substrate. Among them, the coating method is practically preferable because the manufacturing process becomes easy. The coating method is not particularly limited, and examples thereof include an application method, a spray method, and a dip method. For example, the coating method by the application method can be performed with simple operations, for example, by immersing a substrate in a coating solution in which the antithrombogenic material of the present invention is dissolved in an appropriate organic solvent such as alcohol, chloroform, acetone, tetrahydrofuran or dimethylformamide, then removing the extra solution and performing air drying. It is also preferred that heat is applied to the substrate for drying after coating. By this, the adhesion between the substrate and the antithrombogenic material of the present invention can be increased, and the antithrombogenic material can be more firmly fixed.

The organic solvent is selected from ones that do not harm a medical device, that is the substrate, as much as possible. Specific examples thereof include methanol, ethanol, isopropyl alcohol, normalpropyl alcohol, acetone, normalhexane, cyclohexane, tetrahydrofuran, 1,4-dioxane, cyclohexanone, N,N-dimethylformamide, N,N-dimethylacetamide, and N-methylpyrrolidone. Among these, methanol, ethanol, and isopropyl alcohol, which have a low boiling point and are easy for drying after coating, are more preferred.

The (meth)acrylate copolymer according to the present invention obtained by copolymerizing an alkyl (meth)acrylate and/or a silicone (meth)acrylate and a methoxy polyethylene glycol (meth)acrylate can be suitably used as a blood compatible material because the hydrophilicity and hydrophobicity are moderately balanced. In particular, the (meth)acrylate copolymer containing a specific range of silicone (meth)acrylates can suppress the adsorption and adhesion of blood proteins or the like, and thus can be suitably used as a treating material for medical devices and artificial organs. The (meth)acrylate copolymer according to the present invention can be used singly or in combination of two or more.

It is believed that, when the medical device treated with the present antithrombogenic material is in contact with the blood, the highly hydrophilic methoxy polyethylene glycol (meth)acrylate spreads to the surface to exhibit antithrombogenicity, and the hydrophobic (meth)acrylate stays in the vicinity of the substrate to prevent direct contact between the medical device and the blood.

Examples of the method for confirming the durability of the present antithrombogenic material to elution into blood include an alcohol immersion treatment at room temperature. In the alcohol immersion treatment, it is preferred to use a mixed solvent of methanol and ethanol. Since the mixed solvent of methanol and ethanol at a mass ratio of 80/20 is slightly stronger than the elution power of blood, the persistence of antithrombogenicity can be evaluated by immersing the antithrombogenic material in the mixed solvent for 16 hours.

In the present invention, the (meth)acrylate copolymer has the property of not being dissolved in methanol but dissolved in ethanol. The durability (persistence of antithrombogenicity) after 30 days of the blood contact at 37°C can be confirmed even in a short time of 16 hours when methanol and ethanol are mixed at a predetermined ratio as an alcohol immersion treatment solution to confirm the durability after 30 days of blood contact at 37°C. The mass ratio of methanol and ethanol in the alcohol immersion treatment solution is preferably 90-60 : 10-40, more preferably 90-70 : 10-30. In the test using an evaluation sheet described later, it can be determined that the durability is sufficient when the blood clot adhesion is small, that is, the number of evaluation sheets in which the number of blood clot adhesion is observed after immersion in the solvent is 1 or less out of 10 sheets, and the remaining amount of the (meth)acrylate copolymer is 0.1 $\mu g/cm^2$ or more.

In the present invention, one example of the blood compatibility evaluation method of (meth)acrylate is a blood coagulation test. Specifically, it utilizes a reaction in which the fibrin in plasma is gelled by calcium ions and becomes a

fibrin gel. The blood compatibility of the polymer can be confirmed in calcium ion-added plasma in contact with the sample by confirming the presence or absence of blood clot adhesion after immersion in water. Less blood clot adhesion means higher blood compatibility. In the evaluation using an evaluation sheet described later, it can be determined that blood compatibility is good when the number of evaluation sheets in which blood clot adhesion is observed is 4 or less out of 10 sheets.

In the present invention, one example of the method of calculating the coating amount of the sample is quantitation by NMR. Specifically, it is a method of extracting the substrate on which the sample is coated with ethanol, immobilizing the extract solution by drying, then measuring by NMR to calculate the coating amount from the area of the corresponding peak. The durability can be evaluated by comparing the coating amount before and after the alcohol immersion treatment. When the residual amount of the (meth)acrylate copolymer after immersion of the evaluation sheet in 99.5 mass% ethanol is 3.0 $\mu g/cm^2$ or more, it can be determined that it is capable of sufficiently exhibiting the antithrombogenicity at the beginning of the blood contact.

A medical device coated in at least a portion of the surface with the antithrombogenic material according to the present invention can exhibit superior antithrombogenicity. Examples of such medical devices include a blood filter, a blood storage vessel, a blood circuit, an indwelling needle, a catheter, a guidewire, a stent, an artificial lung device, a dialysis device, an antiadhesive material, a wound dressing material, an adhesive for biological tissues, and a repair material for biological tissue regeneration. In particular, a medical device having an extracorporeal circuit and having a blood contact part therein is a preferred aspect of the medical device.

The substrate of the medical device here includes all conventionally used materials. That is, examples thereof include polyvinyl chloride, polycarbonate, polyethylene terephthalate, polyethylene, polypropylene, poly-4-methylpenten-1, thermoplastic polyether polyurethane, thermosetting polyurethane, a silicone rubber such as polydimethylsiloxane having a cross-linked portion, polymethylmethacrylate, polyvinylidene fluoride, polyethylene tetrafluoride, polysulfone, polyether sulfone, polyacetal, polystyrene, an ABS resin and a mixture of these resins, and metals such as stainless steel, titanium, and aluminum. The antithrombogenic material of the present invention balances the material composition, molecular weight, viscosity, and the like, and the coating conditions are optimized. It thus enables uniform and firm coating regardless of the material to which the coating is applied, the shape, the surface properties, or the like.

In the present invention, the method for making the antithrombogenic material carried on a medical device is not particularly limited, but examples include a method of immersing a medical device in a treatment solution in which the antithrombogenic material is dissolved or dispersed in an organic solvent or the like, then removing the solvent by heating or the like. The concentration of the (meth)acrylate copolymer in the treatment solution is preferably 0.001 mass% or more and 10 mass% or less. When the concentration of the (meth)acrylate copolymer is too low, the performance may not be sufficiently exhibited, for example, when applied to a medical device. It is thus more preferred that the concentration is 0.01 mass% or more. When the concentration is too high, there is a risk that the viscosity of the solution increases too much and the workability may be reduced. It is thus preferred that the concentration is 5 mass% or less.

## Examples

Hereinafter, the present invention is described in detail with Examples, but the present invention is not limited by these.

### (Evaluation of alcohol solubility)

To a 50 mL vial, 1 g of the sample was added, then 99 g of ethanol was added, and they were sufficiently mixed. After 30 minutes, the dissolution was visually confirmed. When the ethanol insoluble matters could not be seen visually, it was determined to be ◦ (good). When the ethanol insoluble matters could be seen visually, it was determined to be × (poor).

### (Evaluation of water-insolubility)

100 g of water was added to the solution determined to be alcohol solubility ◦ (good), and they were mixed. When white turbidity remained even after the mixing was continued for 30 minutes, it was determined to be ◦ (good). When white turbidity did not remain after the mixing was continued for 30 minutes, it was determined to be × (poor).

### (Measurement of weight-average molecular weight)

15 mg of the sample was weighed into a vial, and 3.0 mL of the mobile phase for GPC measurement was added, then the mixture was left still overnight. The solution was filtered with a 0.45 $\mu m$ hydrophilic PTFE membrane filter cartridge (Millex-LH, Nihon Millipore K.K.). For GPC measurement, a 515 HPLC pump and a 717plus automatic

injection device (Nihon Waters K.K.) were used, and 2 × PLgel 5 μMIXED-D, 7.5 × 300 mm (Agilent Technologies, Inc.) were used as the column. The column temperature was 40°C, and the mobile phase was tetrahydrofuran for liquid chromatography (Fujifilm Wako Pure Chemical Corporation) containing dibutyl hydroxytoluene as a stabilizer. Detection was performed with RI and the injection amount was 20 μL. The molecular weight calibration was performed with monodispersed polystyrene (Easi Cal PS-1, Agilent Technologies, Inc.).

(Measurement of reduced viscosity)

In a glass bottle, 1 g of the sample was weighed, and 15 mL of acetone (Fujifilm Wako Pure Chemical Corporation) was added. They were shaken and mixed by hand once every 20 minutes. After 1 to 2 hours, the dissolution was visually confirmed, and the mixture was transferred to a 25 mL volumetric flask. After co-washing, acetone was added to prepare 25 mL of solution. Subsequently, it was filtered with a 5 μm diameter filter (Merck) to obtain a test solution. The falling time of the sample solution and acetone at 30°C was measured using an Ubbelohde viscometer with a viscometer constant C = 0.003426 (cSt/s), and the reduced viscosity was calculated by the following Equation 1:

$$(A/B - 1)/C = (A/B - 1)/(S/25 \times 100) = (A/B - 1)/(S \times 4)$$

A: Falling time in seconds (seconds) of sample solution
B: Falling time in seconds (seconds) of acetone
S: Specimen weight (g)
C: Sample solution concentration (g/dL)

(Measurement of mass reduction)

The weighing bottle was placed in an electric furnace set at 105°C and dried for 30 minutes. Thereafter, it was allowed to cool in a desiccator for 20 minutes, and the mass of the weighing bottle was measured. 0.5 g of the sample was weighed in a weighing bottle. Similarly, the weighing bottle was placed in an electric furnace set at 105°C and dried for 2 hours. Thereafter, it was allowed to cool in a desiccator for 20 minutes, and the mass of the weighing bottle was measured. Mass reduction (%) was calculated from the obtained mass of each weighing bottle. When the mass reduction (%) was 5% or less, it was determined that the sample was sufficiently dried.

(Measurement of residual monomer content)

0.2 g of the sample was dissolved in 2 mL of acetone (Fujifilm Wako Pure Chemical Corporation), then the residual monomer was quantified by GC measurement after appropriate dilution. The measuring device used was GC-2010 Plus (SHIMADZU CORPORATION), and the column used was Rtx-5 (GL Sciences Inc.). The inlet temperature was set to 150°C, the detector temperature to 280°C, and the oven temperature to 40°C. When the residual monomer content was 4,000 ppm or less, it was determined that purification was sufficiently performed.

(Measurement of copolymerization composition ratio)

20 mg of the sample was dissolved in 1 mL of heavy chloroform, and $^1$H-NMR measurement was performed with a 400 MHz superconductivity Fourier transform nuclear magnetic resonance device (400-MR, Agilent Technologies, Inc.).

(Preparation of evaluation sheet)

Ethanol solutions of each concentration were prepared by adding ethanol to the sample to be a total of 100 g and dissolving it, and they were used as treatment solutions. The concentrations prepared are each sample concentration shown in Table 2. After immersing the half surface portion (2 cm × 2 cm × 0.1 cm) of a polycarbonate sheet (4 cm × 2 cm × 0.1 cm) in the treatment solution for 10 seconds, the polycarbonate sheet was removed and dried on both sides with air at a flow rate of 10 mL/min for 30 seconds each. Then, it was dried at room temperature for 16 hours to obtain an evaluation sheet.

(Blood coagulation test)

800 μL of preserved rabbit blood (model number: 003-00053-01 JAPAN BIO SERUM CO., LTD.) was added

to a 15 mL of centrifuge tube. A 66.6 μL of 80 mM calcium chloride solution was added, and the mixture was stirred well, which was taken as a test blood solution. The evaluation sheet was placed on a plastic dish. The plastic dish was placed in a water bath set at 37°C. 200 μL of the test blood solution was added to the untreated portion and the treated portion of the evaluation sheet. Subsequently, it was incubated at 37°C for 25 minutes. The evaluation sheet after incubation was submerged in 100 mL of physiological saline (Otsuka Pharmaceutical Co., Ltd.) and shaken lightly. The evaluation sheet was pulled up from the physiological saline, and the number of blood clot adhesion on the surface of the evaluation sheet was checked. When the number of evaluation sheets in which blood clot adhesion was observed was 4 or less out of 10 sheets, it was determined that the blood compatibility was good.

(Coating quantification test)

The evaluation sheet was immersed in 5 mL of ethanol (99.5 mass%) for 30 minutes to obtain an extract solution. The obtained extract solution was sprayed with nitrogen gas at 40°C and solidified by drying until the ethanol odor disappeared. This procedure was repeated 3 times. To the whole amount of the obtained dry solids, 0.13 mg of dimethyl isophthalate was added, and then the mixture was dissolved in 0.6 mL of deuterated chloroform, and $^1$H-NMR measurement was performed at 30°C. The coating amount was calculated from the obtained peak area. The coating amount was calculated using the following Equation.

$$\text{Coating amount} = C \times 218.023 \times B \times 1000000/19400/A$$

A: Sample weight
B: Dimethyl isophthalate weight
C: Integral value of peaks derived from methoxy triethylene glycol acrylate when the integral value of peaks derived from 1,3-dimethyl-2-imidazolidinone is set to 100

(Alcohol immersion treatment test)

The evaluation sheet was immersed in 150 mL of alcohol immersion treatment solution (methanol : ethanol = 80 mass% : 20 mass%) for 16 hours. After immersion, the evaluation sheet was well dried and subjected to the blood coagulation test and coating quantification in the same manner as described above.

(Example 1)

To 471.8 g of methoxy triethylene glycol acrylate (MTEGA) (SHIN-NAKAMURA CHEMICAL Co., Ltd.), 78.0 g of silicone methacrylate (PDMSMA) (Gelest Inc., product name; MCR-M11) and 694.5 g of 2-ethylhexyl acrylate (EHA) (TOAGOSEI CO., LTD.), 1.2325 g of azobisisobutyronitrile (AIBN) (Fujifilm Wako Pure Chemical Corporation) was added, and the polymerization reaction was carried out in 1628.3 g of ethanol (KISHIDA CHEMICAL CO., LTD.) at 85°C for 3 hours. After the polymerization reaction was completed, the resultant was dried at normal pressure at 85°C for 2 hours. Then, it was dried under reduced pressure at 60°C for 1 hour to obtain a concentrate. The concentrate was divided into two equal parts concentrate A and concentrate B. To 612.1 g of concentrate A, 3162.7 g of methanol (KISHIDA CHEMICAL CO., LTD.) and 305.3 g of water were then added, and the mixture was stirred for 30 minutes. After completion of the stirring, the mixture was left still for 1.5 hours, and the supernatant was removed by decantation. To this precipitate, methanol was added, and the mixture was stirred for 30 minutes, and left still for 1.5 hours, then the supernatant was removed by decantation. This process was repeated three times in the same manner to obtain precipitation A. The added amount of methanol used was 3161.7 g, 3161.4 g, and 3163.9 g, in order. Similarly, 3162.3 g of methanol and 303.1 g of water were added to 614.4 g of concentrate B, and the mixture was stirred for 30 minutes. After completion of the stirring, the mixture was left still for 1.5 hours, and the supernatant was removed by decantation. To this precipitate, methanol was added, and the mixture was stirred for 30 minutes, and left still for 1.5 hours, then the supernatant was removed by decantation. This process was repeated three times in the same manner to obtain precipitate B. The added amount of methanol used was 3165.5 g, 3167.5 g, and 3165.4 g, in order. The obtained precipitate A and precipitate B were combined into one, and dried under reduced pressure under the conditions of 40°C for 1.5 hours to obtain copolymer 1.

(Example 2)

To 94.25 g of methoxy triethylene glycol acrylate (MTEGA) (SHIN-NAKAMURA CHEMICAL Co., Ltd.), 15.59 g of silicone methacrylate (PDMSMA) (Gelest Inc., product name; MCR-M11) and 138.68 g of 2-ethylhexyl acrylate

(EHA) (TOAGOSEI CO., LTD.), 0.2469 g of azobisisobutyronitrile (AIBN) (Fujifilm Wako Pure Chemical Corporation) was added, and the polymerization reaction was carried out in 165.18 g of ethanol (KISHIDA CHEMICAL CO., LTD.) under the conditions of 85°C for 3 hours. After the polymerization reaction was completed, the resultant was dried at normal pressure under the conditions of 85°C for 1 hour. Then, it was dried under reduced pressure at 60°C for 1.5 hours to obtain a concentrate. The concentrate was divided into two equal parts concentrate A and concentrate B. To 122.51 g of concentrate A, 631.97 g of methanol (KISHIDA CHEMICAL CO., LTD.) was then added, and the mixture was stirred at 60°C for 30 minutes. After completion of the stirring, the mixture was left still for 1.5 hours, and the supernatant was removed by decantation. To this precipitate, methanol was added, and the mixture was stirred at 60°C for 30 minutes, and left still for 1.5 hours, then the supernatant was removed by decantation. This process was repeated three times in the same manner to obtain precipitate A. The added amount of methanol used was 631.98 g, 632.03 g, and 632.05 g, in order. Similarly, 631.99 g of methanol was added to 122.50 g of concentrate B, and the mixture was stirred at 60°C for 30 minutes. After completion of the stirring, the mixture was left still for 1.5 hours, and the supernatant was removed by decantation. To this precipitate, methanol was added, and the mixture was stirred at 60°C for 30 minutes, and left still for 1.5 hours, then the supernatant was removed by decantation. This process was repeated three times in the same manner to obtain precipitate B. The added amount of methanol used was 631.98 g, 632.00 g, and 632.01 g, in order. The obtained precipitate A and precipitate B were combined into one, and dried at normal pressure under conditions of 85°C for 30 minutes, and then dried under reduced pressure under the conditions of 40°C for 1 hour to obtain copolymer 2.

(Comparative Example 1)

To 94.25 g of methoxy triethylene glycol acrylate (MTEGA) (SHIN-NAKAMURA CHEMICAL Co., Ltd.), 15.59 g of silicone methacrylate (Gelest Inc., product name; MCR-M11) and 138.68 g of 2-ethylhexyl acrylate (EHA) (TOAGOSEI CO., LTD.), 0.247 g of azobisisobutyronitrile (AIBN) (Fujifilm Wako Pure Chemical Corporation) was added, and the polymerization reaction was carried out in 165.18 g of ethanol (Fujifilm Wako Pure Chemical Corporation) under the conditions of 85°C for 3 hours. After the polymerization reaction was completed, the resultant was dried at normal pressure under the conditions of 85°C for 1 hour. Then, it was dried under reduced pressure under the conditions of 60°C for 1.5 hours to obtain a concentrate. To the concentrate, 696.35 g of methanol (Fujifilm Wako Pure Chemical Corporation) was added, and the mixture was stirred for 30 minutes. After completion of the stirring, the mixture was left still for 1 hour, and the supernatant was removed by decantation. The precipitate was dried at normal pressure under the conditions of 85°C for 30 minutes, and then dried under reduced pressure under the conditions of 40°C for 1 hour to obtain copolymer 3.

(Comparative Example 2)

To 94.27 g of methoxy triethylene glycol acrylate (MTEGA) (SHIN-NAKAMURA CHEMICAL Co., Ltd.), 15.60 g of silicone methacrylate (PDMSMA) (Gelest Inc., product name; MCR-M11) and 138.66 g of 2-ethylhexyl acrylate (EHA) (TOAGOSEI CO., LTD.), 0.2465 g of azobisisobutyronitrile (AIBN) (Fujifilm Wako Pure Chemical Corporation) was added, and the polymerization reaction was carried out in 580.03 g of ethanol (KISHIDA CHEMICAL CO., LTD.) under the conditions of 85°C for 3 hours. After the polymerization reaction was completed, the resultant was dried at normal pressure under the conditions of 85°C for 1 hour. Then, it was dried under reduced pressure under the conditions of 60°C for 1.5 hours to obtain a concentrate. The concentrate was divided into two equal parts concentrate A and concentrate B. To 122.53 g of concentrate A, 632.00 g of methanol (KISHIDA CHEMICAL CO., LTD.) was then added, and the mixture was stirred at room temperature for 30 minutes. After completion of the stirring, the mixture was left still for 1.5 hours, and the supernatant was removed by decantation. To this precipitate, methanol was added, and the mixture was stirred at room temperature for 30 minutes, and left still for 1.5 hours, then the supernatant was removed by decantation. This process was repeated three times in the same manner to obtain precipitate A. The added amount of methanol used was 632.09 g, 632.09 g, and 632.32 g, in order. The obtained precipitate A was dried at normal pressure under the conditions of 85°C for 30 minutes, and then dried under reduced pressure under the conditions of 40°C for 1 hour to obtain copolymer 4.

As shown in Tables 1 and 2, when the copolymer of Examples with high reduced viscosity and weight-average molecular weight was used to make an evaluation sheet, good results were obtained in the blood coagulation test after the alcohol immersion treatment. It is presumed that, since the copolymer of Examples has a greater reduced viscosity than the conventional (meth)acrylate copolymer, the thickness of the coating becomes more uniform. The mechanism is shown in Table 3.

In other words, when the copolymer of Examples coats, water in the air that adheres to the top surface of the coating is delocalized upon ethanol removal, and thus the thickness of the coating becomes uniform. It is presumed that this is a result that the development of uncoated areas (defects such as coating spots) in the subsequent alcohol

immersion treatment can be suppressed. It thus has been found that the present antithrombogenic material has sufficient durability to elution into the blood.

[Table 1]

| | Example 1 | Example 2 | Comparative Example 1 | Comparative Example 2 |
|---|---|---|---|---|
| Alkyl (meth)acrylate | EHA | EHA | EHA | EHA |
| Number of dimethylsiloxane repeat units in silicone (meth)acrylate | 11 | 11 | 11 | 11 |
| Number of PEG chain repeat units in methoxy polyethylene glycol (meth) acrylate | 3 | 3 | 3 | 3 |
| Alcohol-solubility | ○ | ○ | ○ | ○ |
| Water-insolubility | ○ | ○ | ○ | ○ |
| Reduced viscosity of copolymer (dl/g) | 0.214 | 0.350 | 0.284 | 0.171 |
| Weight average molecular weight of (meth) acrylate copolymer | 68,300 | 133,000 | 113,000 | 46,700 |
| Mass reduction (%) | 0.17 | 0.07 | 0.58 | 0.13 |
| Residual monomer measurement (ppm) | <40 | 178 | 5,300 | 237 |
| Alkyl (meth)acrylate composition ratio (mol%) | 67.4 | 66.5 | 66.1 | 68.3 |
| Silicone (meth)acrylate composition ratio (mol%) | 1.7 | 1.6 | 1.4 | 1.6 |
| Methoxy polyethylene glycol (meth) acrylate composition ratio (mol%) | 30.9 | 31.9 | 32.5 | 30.1 |

[Table 2]

| | Example 1 | | | | | Example 2 | | | Comparative Example 1 | | | | Comparative Example 2 | | | |
|---|---|---|---|---|---|---|---|---|---|---|---|---|---|---|---|---|
| Coating concentration (%) | 0.3 | 0.5 | 1.0 | 3.0 | 4.0 | 0.1 | 0.3 | 0.5 | 0.1 | 0.3 | 0.5 | 3.0 | 0.1 | 0.3 | 0.5 | 3.0 |
| Coating quantification ($\mu g/cm^2$) | 3.5 | 7.9 | 11.7 | 41.7 | 78.7 | | | | 1.3 | 4.4 | 6.1 | 54.8 | | | | |
| Blood coagulation test (Number of blood clot adhesion specimens) | 0 | 0 | 0 | 0 | 0 | 0 | 0 | 0 | 0 | 0 | 0 | 0 | 0 | 0 | 0 | 0 |
| Coating quantification after alcohol immersion treatment ($\mu g/cm^2$) | 0.1 | 0.7 | 0.3 | 1.3 | 19.2 | 0.1 | 0.2 | 1.0 | <0.1 | 0.1 | 0.3 | 2.1 | | | | |
| Blood coagulation test after alcohol immersion treatment (Number of blood clot adhesion specimens) | 3 | 4 | 1 | 3 | 0 | 0 | 0 | 0 | 2 | 1 | 1 | 0 | 10 | 10 | 9 | 6 |

[Table 3]

| Process | Comparative Example | Example |
|---|---|---|
| Application of treatment solution | A treatment solution is uniformly applied to the top surface of the polycarbonate (PC) sheet | ← |
| Ethanol removal | When ethanol in the treatment solution is volatilized, the temperature on the top surface of the PC sheet decreases, and water in the air cooled adheres to the top surface of the PC sheet | ← |
| Water fixation | Due to the low reduced viscosity of the copolymer, water with a specific gravity greater than ethanol pushes away the copolymer and water is localized | Due to the high reduced viscosity of the copolymer, water with a specific gravity greater than ethanol does not push away the copolymer and water is not localized |
| Removal of water | The copolymer coating thickness of the part where the localized water on the top surface of the PC sheet has been volatilized is partially thinned, resulting in non-uniform thickness of the copolymer coating | Delocalized water on the top surface of the PC sheet is removed, resulting in uniform thickness of the copolymer coating |
| During alcohol immersion treatment | The copolymer uniformly flows out of the PC sheet in the thickness direction | ← |
| After alcohol immersion treatment | Copolymer uncoated areas are partially developed, failing to exhibit antithrombogenicity | No copolymer uncoated areas occurred, allowing to exhibit antithrombogenicity |

In the present invention, it has been found that the present antithrombogenic material exhibits good blood compatibility even after alcohol immersion treatment at room temperature for 16 hours. As a result, it has been shown that the present antithrombogenic material can maintain blood compatibility for a longer period compared to conventional antithrombogenic materials.

INDUSTRIAL APPLICABILITY

The copolymer according to the present invention has excellent blood compatibility and can be used as a material with high hydrophilicity. Since the physical properties of the material are viscous and insoluble in water, the present invention can provide a material that can coat throughout the blood circuit without compromising the physical properties of medical equipment. Thus, the present invention will largely contribute to the development of the industry.

**Claims**

1. An antithrombogenic material comprising a (meth)acrylate copolymer having a weight-average molecular weight of 50,000 or more and 1,500,000 or less, the (meth)acrylate copolymer being obtained by copolymerizing an alkyl (meth)acrylate unit represented by the following Formula 1, a silicone (meth)acrylate unit represented by the following Formula 2, and a methoxy polyethylene glycol (meth)acrylate unit represented by the following Formula 3 at a molar ratio of 80-20/10-0.01/10-79.99, wherein the (meth)acrylate copolymer is water-insoluble, the water-insoluble means that, when the (meth)acrylate copolymer is left still for 30 days in a physiological saline at 37 °C at 99 mass% with respect to 1 mass% of the copolymer, the mass reduction rate of the copolymer is 1 mass% or less, the (meth)acrylate copolymer is a viscous liquid at room temperature, the (meth)acrylate copolymer is soluble in any of alcohols having 1 to 6 carbon atoms, a residual monomer content is 4,000 ppm or less, and a reduced viscosity ($\eta sp/c$) is 0.18 dl/g or more and 3.00 dl/g or less,

[Formula 1]

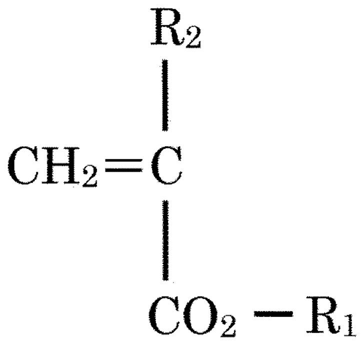

[1]

wherein, $R^1$ represents an alkyl group having 8 to 12 carbon atoms, $R^2$ represents a hydrogen atom or a methyl group,

[Formula 2]

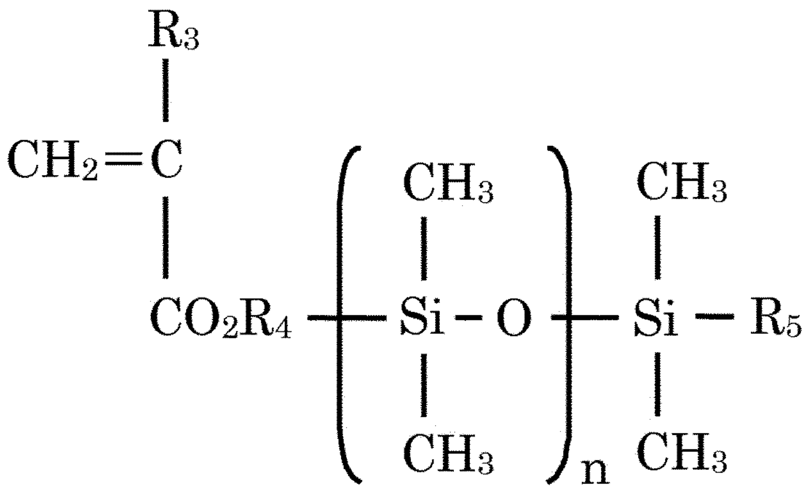

[2]

wherein, $R^3$ is a hydrogen atom or a methyl group, $R^4$ is an alkylene group having 1 to 6 carbon atoms, $R^5$ is an alkyl group having 1 to 6 carbon atoms, and n is an integer of 1 to 30,

[Formula 3]

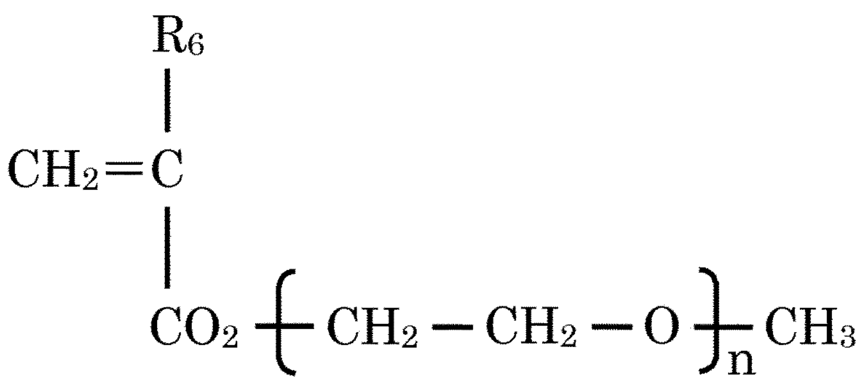

[3]

wherein $R^6$ represents a hydrogen atom or a methyl group, and n represents an integer of 2 to 4.

**2.** A medical device comprising the antithrombogenic material according to claim 1.

**INTERNATIONAL SEARCH REPORT**

International application No.
**PCT/JP2022/015625**

**A. CLASSIFICATION OF SUBJECT MATTER**

***A61L 33/06***(2006.01)i; ***C08F 222/10***(2006.01)i; ***C08F 230/08***(2006.01)i
FI: A61L33/06 200; C08F222/10; C08F230/08

According to International Patent Classification (IPC) or to both national classification and IPC

**B. FIELDS SEARCHED**

Minimum documentation searched (classification system followed by classification symbols)

A61L33/06; C08F222/10; C08F230/08

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

**C. DOCUMENTS CONSIDERED TO BE RELEVANT**

| Category* | Citation of document, with indication, where appropriate, of the relevant passages | Relevant to claim No. |
|---|---|---|
| Y | JP 2009-261437 A (TOYO BOSEKI) 12 November 2009 (2009-11-12) claims 1, 2, 4, paragraphs [0001], [0055], [0065]-[0071], [0089], synthesis example 4, table 1 | 1, 2 |
| Y | WO 2019/142710 A1 (TERUMO CORP) 25 July 2019 (2019-07-25) claims 1, 8, paragraph [0023], examples | 1, 2 |
| Y | JP 2002-356519 A (NOF CORP) 13 December 2002 (2002-12-13) claims, paragraphs [0050], [0065], examples | 1, 2 |
| A | JP 2008-289864 A (TOYO BOSEKI) 04 December 2008 (2008-12-04) entire text | 1, 2 |
| A | JP 2008-266225 A (TOYO BOSEKI) 06 November 2008 (2008-11-06) entire text | 1, 2 |

☐ Further documents are listed in the continuation of Box C.　☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **07 June 2022** | **14 June 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2022/015625**

| Patent document cited in search report | Publication date (day/month/year) | Patent family member(s) | Publication date (day/month/year) |
|---|---|---|---|
| JP 2009-261437 A | 12 November 2009 | (Family: none) | |
| WO 2019/142710 A1 | 25 July 2019 | (Family: none) | |
| JP 2002-356519 A | 13 December 2002 | (Family: none) | |
| JP 2008-289864 A | 04 December 2008 | (Family: none) | |
| JP 2008-266225 A | 06 November 2008 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 4793700 B